Europäisches Patentamt

European Patent Office

Office européen des brevets

(11) Publication number: **0 484 963 B1**

# EUROPEAN PATENT SPECIFICATION

(45) Date of publication of patent specification: **19.07.95**

(51) Int. Cl.6: **C07C 67/03**, C07C 69/82, C07C 31/20, C07C 29/09

(21) Application number: **91119061.9**

(22) Date of filing: **08.11.91**

(54) **Recovery of methyl esters of aromatic acids and glycols from thermoplastic polyester scrap.**

(30) Priority: **09.11.90 US 610325**

(43) Date of publication of application:
**13.05.92 Bulletin 92/20**

(45) Publication of the grant of the patent:
**19.07.95 Bulletin 95/29**

(84) Designated Contracting States:
**BE DE ES FR GB IT NL**

(56) References cited:
**CH-A- 425 761**
**NL-A- 273 455**
**US-A- 5 051 528**

(73) Proprietor: **E.I. DU PONT DE NEMOURS AND COMPANY**
**1007 Market Street**
**Wilmington**
**Delaware 19898 (US)**

(72) Inventor: **Michel, Robert Everette**
**218 Cabbage Inlet Lane**
**Wilmington,**
**North Carolina 28403 (US)**

(74) Representative: **Abitz, Walter, Dr.-Ing. et al**
**Patentanwälte Abitz & Partner**
**Postfach 86 01 09**
**D-81628 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

## FIELD OF THE INVENTION

This invention relates to the recovery of methyl esters of aromatic acids such as dimethyl terephthalate and glycols such as ethylene glycol from thermoplastic polyester scrap by treating the scrap with excess methanol vapor at high temperatures.

## BACKGROUND OF THE INVENTION

Processes for the recovery of dimethyl terephthalate and ethylene glycol from waste polyester are known. See, for example, U.S. Patents 3,776,945, U.S. Patent 3,488,298 and U.S. Patent 2,884,443. In the '945 patent polyester waste is treated with methanol to form dimethyl terephthalate, the excess methanol is then vaporized and then the dimethyl terephthalate and ethylene glycol are vaporized. The '298 patent depolymerizes polyester by treating it with methanol, then adding phosphorus compound, and finally vaporizing the methanol, ethylene glycol and dimethyl terephthalate. The '443 patent depolymerizes polyester by heating the polyester to a temperature below 210°C in the presence of methanol, a re-esterification catalyst, and a high boiling organic liquid. The dimethyl terephthalate is separated as crystals from the reaction vessel.

## SUMMARY OF THE INVENTION

The process of the present invention is for the recovery of dimethyl terephthalate from polymers of terephthalic acid and a glycol, which comprises treating said polymers in a reaction zone with methanol vapor at a temperature above 230 degrees C and at a pressure below 15.2 bar (15 atmospheres), continuously removing vapors of methanol, dimethyl terephthalate, and the glycol from the reaction zone, said vapors containing at least 3 moles of methanol for every mole of dimethyl terephthalate, separating methanol from said vapors, and separating dimethyl terephthalate from the vapors.

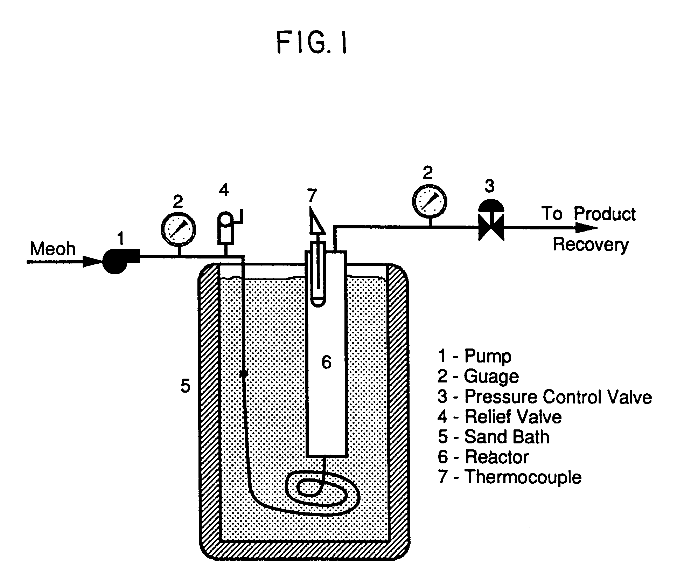

## DESCRIPTION OF THE DRAWING

The drawing is a view, partly in cross-section of a reactor suitable for carrying out the process of the invention.

## DETAILED DESCRIPTION

The present invention is based on the discovery that polyester resin can be depolymerized by methanol vapor heated to above the critical temperature, i.e. 230°C and that the resulting monomers, dimethyl terephthalate and the glycol, usually ethylene glycol, can be swept continuously from the reactor as vapors along with methanol vapor if the methanol is present in the vapors removed from the reactor in an amount of at least 3 moles of methanol for every mole of dimethyl terephthalate. (The term "critical temperature" is used in its usual technical sense: that is 230°C in the temperature at which it is not possible to liquify methanol by increasing the pressure). The vapors from the reactor are then separated by condensation in a separation column, or cooled and the crystallized dimethyl terephthalate separated, for example, by filtration.

The excess methanol vapor fed to the reactor, aids in the removal of the dimethyl terephthalate vapor from the reactor by acting as a carrier gas. It is preferred that the vapor leaving the reactor contain at least 5 moles of methanol per mole of dimethyl terephthalate, and mole ratios as high as 100 moles of methanol to one mole of dimethyl terephthalate have been demonstrated as useful.

In addition to polyester containing ethylene glycol, polymers containing units of butylene glycol can be depolymerized and the monomers recovered by the process of this invention.

It is not necessary that the polyester polymer that is treated by the present process be free from contaminants. The process works well on blend of polyester fibers with other fibers such as cotton, polyester that is metallized, polyester that is dyed, polyester that is pigmented, polyester that is mixed with other plastics, etc.

If desired the reactor may also contain solvent for the polyester. As shown in Run 13 below, a suitable solvent is a mixture of methylhydrogen-terephthalate and dimethyl terephthalate. Other solvents such as diphenyl, diphenyl ether, diphenylmethane may make it possible to run the process efficiently at somewhat lower temperatures than the temperature employed if no solvent is employed. In the absence of solvent the temperature of the reactor is usually maintained above the melting point of the polyester. The temperature is in any case always above the critical temperature for methanol, of 230°C. The pressure in the reaction vessel is usually maintained between 1.01 bar and 15.2 bar (1 and 15 atmospheres). Pressures of 4.04 and 10.1 bar (4 to 10 atmospheres) are preferred.

The process does not require the use of depolymerization catalysts, and thus avoids the introduction of one or more ingredients that would subsequently have to be removed or neutralized if the monomers were to be again polymerized.

## EXAMPLES

The depolymerizations were carried out in the apparatus shown in the Figure. The reactor 6 was charged with the polyester shown in the table and placed in a pre-heated sand bath 5. Methanol flow was started via pump 1. The pressure was measured using gauges 2. The temperature measured by thermocouple 7. The incoming line had a pressure relief valve 4, and the exit line had a pressure control valve 3. At the end of the run the total reactor effluent was weighed and the ester and available glycol content determined. The reactor was cooled, dissembled and the amount of residue determined. The results of the depolymerization of polyethylene terephthalate from varied sources is shown in the table.

**TABLE**

| Source of poly(ethyleneglycol) terephthalate (PET) | RUN | TEMP. (C) | PRES. (bar(PSIG)) | MHET(2) | PRODUCT (1) DMT(3) | EG(4) |
|---|---|---|---|---|---|---|
| Co-mingled green bottle waste (80% PET, 20% high density polyethylene) | 1 | 260 | 6.53(80) | 2 | 75 | 80 |
|  | 2 | 280 | 7.22(90) | 1 | 85 | 88 |
| Floor clippings, (60-40 PET-cotton) | 3 | 260 | 8.59(110) | 4 | 83 | 92 |
|  | 4 | 280 | 7.91(100) | 2 | 82 | 89 |
|  | 5 | 300 | 7.56(95) | 4 | 88 | 90 |
| Magnetic tape containing iron and chromium oxides | 6 | 260 | 8.59(110) | 5 | 88 | 90 |
|  | 7 | 280 | 7.91(100) | 5 | 85 | 85 |
| Polyvinylidine chloride coated PET film | 8 | 260 | 5.49(65) | 6 | 84 | 90 |
|  | 9 | 300 | 5.15(60) | 8 | 82 | 90 |
| Metallized PET film | 10 | 270 | 5.15(60) | 4 | 86 | 80 |
| Photoresist coated PET | 11 | 280 | 5.49(65) | 7 | 84 | 91 |
| PET Oligomer having a degree of polymerization of 2. | 12 | 260 | 6.87(85) | 6 | 84 | 94 |
| Clear PET and 25% solvent(5) | 13 |  |  |  | 95(6) | 88(7) |

(1) Per-cent yields based on PET content of the starting material.
(2) Methyl(2-hydroxyethyl)terephthalate
(3) Dimethylterephthalate
(4) Ethylene glycol
(5) 20% Methyl hydrogenterephthalate and 80% dimethylterephthalate.
(6) Based on Total Terephthalate.
(7) Based on PET.

## Claims

1. A process for the recovery of dimethyl terephthalate from polymers of polyolefin terephthalate, which comprises treating said polymers in a reaction zone with methanol vapor at a temperature above 230 degrees C and at a pressure below 15.2 bar (15 atmospheres), continuously removing vapors of methanol, dimethyl terephthalate, and an olefin glycol from the reaction zone, said vapors containing at least 3 moles of methanol for every mole of dimethyl terephthalate, separating methanol from said vapors, and recovering dimethyl terephthalate.

2. The process of claim 1 in which the methanol is separated from the vapors exiting the reaction zone by condensation, and the dimethyl terephthalate is recovered by condensation.

3. The process of claim 1 in which the reaction zone also contains a solvent for the polyolefin terephthalate.

EP 0 484 963 B1

4. The process of claim 1 in which the glycol is ethylene glycol.

5. The process of claim 1 in which the pressure is between 1.01 and 15.2 bar (1 and 15 atmospheres).

6. The process of claim 2 in which the glycol is also recovered by condensation.

**Patentansprüche**

1. Verfahren zur Wiedergewinnung von Dimethylterephthalat aus Polymeren von Polyolefinterephthalat, umfassend die Behandlung der genannten Polymeren in einer Reaktionszone mit Methanoldampf bei einer Temperatur oberhalb 230°C und einem Druck unterhalb 15,2 bar (15 Atmosphären), die kontinuierliche Entfernung der Dämpfe von Methanol, Dimethylterephthalat und einem Olefinglycol aus der Reaktionszone, wobei die genannten Dämpfe wenigstens 3 Mol Methanol pro jedes Mol Dimethylterephthalat enthalten, die Abtrennung des Methanols von den genannten Dämpfen und die Wiedergewinnung von Dimethylterephthalat.

2. Verfahren nach Anspruch 1, bei welchem das Methanol von den die Reaktionszone verlassenden Dämpfen durch Kondensation abgetrennt und das Dimethylterephthalat durch Kondensation wiedergewonnen wird.

3. Verfahren nach Anspruch 1, bei welchem die Reaktionszone auch ein Lösungsmittel für Polyolefinterephthalat enthält.

4. Verfahren nach Anspruch 1, bei welchem das Glycol Ethylenglycol ist.

5. Verfahren nach Anspruch 1, bei welchem der Druck zwischen 1,01 und 15,2 bar (1 und 15 Atmosphären) liegt.

6. Verfahren nach Anspruch 2, bei welchem das Glycol ebenfalls durch Kondensation wiedergewonnen wird.

**Revendications**

1. Un procédé de récupération de diméthyltéréphtalate à partir de polymères de polyoléfine téréphtalate qui comprend le traitement desdits polymères dans une zone réactionnelle avec de la vapeur de méthanol à une température supérieure à 230°C et à une pression inférieure à 15,2 bar (15 atmosphères), en éliminant en continu les vapeurs de méthanol, le diméthyltéréphtalate, et une oléfine glycol de la zone réactionnelle, lesdites vapeurs contenant au moins 3 moles de méthanol pour chaque mode de diméthyltéréphatalate, la séparation du méthanol desdites vapeurs et la récupération du diméthyltéréphtalate.

2. Le procédé selon la revendication 1, dans lequel le méthanol est séparé des vapeurs sortant de la zone réactionnelle par condensation, et le diméthyltéréphtalate est récupéré par condensation.

3. Le procédé selon la revendication 1, dans lequel la zone réactionnelle contient également un solvant pour le polyoléfine téréphtalate.

4. Le procédé selon la revendication 1, dans lequel le glycol est de d'éthylèneglycol.

5. Le procédé selon la revendication 1, dans lequel la pression est comprise entre 1,01 et 15,2 bar (1 et 15 atmosphères).

6. Le procédé selon la revendication 2, dans lequel le glycol est également récupéré par condensation.

5

# FIG. I

Meoh

1 - Pump
2 - Guage
3 - Pressure Control Valve
4 - Relief Valve
5 - Sand Bath
6 - Reactor
7 - Thermocouple

To Product
Recovery